# EUROPEAN PATENT APPLICATION

(11) **EP 1 967 511 A1**
(43) Date of publication of application: **10.09.2008**
(21) Application number: 07103655.2
(22) Date of filing: 07.03.2007
(51) Int. Cl.: C07C 251/24

(54) **Process for the preparation of a benzophenone glycine imine alkyl ester derivative**

(71) Applicant: Bayer CropScience AG, 40789 Monheim (DE)
(72) Inventor: Ressel, Hans-Joachim, 65795, Hattersheim (DE)
(74) Representative: Gerhards, Frank

(57) **Abstract**

Process for the preparation of a benzophenone glycine imine alkyl ester derivative of general formula (I) :

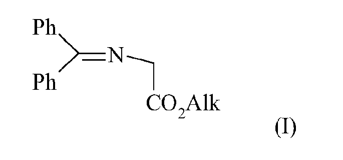

## Description

The present invention relates to a novel process for the preparation of a benzophenone glycine imine alkyl ester derivative starting with a,a-dichlorodiphenylmethane. Benzophenone glycine imine alkyl ester derivative might be useful as an intermediate compound for the preparation of 2-aminomethylpyridine derivative, which is a key intermediate in the preparation of certain pesticides, and the preparation of certain 2-amino acid derivatives..

Patent application WO 99/42447 discloses the preparation of 2-aminomethylpyridines starting from benzophenone glycine imine alkyl ester. This document does not disclose a method for the preparation of benzophenone glycine imine alkyl ester.

Patent application WO 2004/065359 discloses the preparation of benzophenone glycine imine alkyl ester derivatives starting from benzophenone followed by the preparation of 2-aminomethylpyridine derivatives. This document does not disclose a process for the preparation of benzophenone glycine imine alkyl ester derivatives starting with a,a-dichloro-diphenylmethane. Furthermore, the preparation of benzophenone glycine imine alkyl ester derivatives starting from benzophenone presents the drawback in that it requires the presence of a catalyst, which always increases costs and complexity of the process when used at an industrial scale.

Certain methods for the preparation of benzophenone glycine imine alkyl ester derivatives have been reported in Journal of Peptide Research (2000), 55(4), page 300-307*.* This article discloses the reaction of benzophenone imine with an alkyl glycinate hydrochloride to prepare a benzophenone glycine imine alkyl ester. The use of a,a-dichloro-diphenylmethane is not disclosed in that document. The use of benzophenone imine presents the drawback in that it is expensive.

We have now found an alternative method to prepare a benzophenone glycine imine alkyl ester derivative which does not present the above mentioned drawbacks while maintaining a comparable reaction yield to the process disclosed in International patent application WO 2004/065359, which renders this process applicable to industrial scale operation.

Accordingly, the present invention relates to a process for the preparation of a benzophenone glycine imine alkyl ester derivative of general formula (I) : 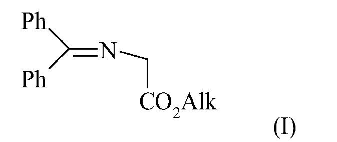 in which :
- Ph represents phenyl; and
- Alk represents a C₁-C₆ alkyl moiety;
according to the following reaction scheme : 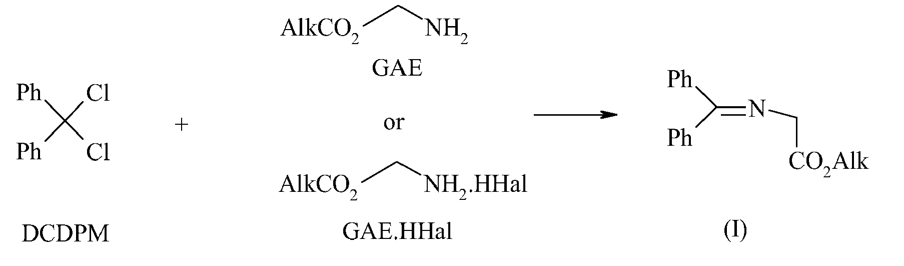 in which a,a-dichloro-diphenylmethane (DCDPM) is reacted with glycine alkyl ester (GAE) or its hydrohalide (GAE.HHal) in a solvent, in the presence of a base, in a DCDPM / GAE or GAE.HHal molar ratio of from 0,5 to 2 and in a DCDPM / base molar ratio of from 1 to 5 to provide a benzophenone glycine imine derivative of general formula (I).

In the context of the present invention :
- Hal means a halogen atom which may be a bromine atom, a chlorine atom, a iodine atom or a fluorine atom; and
- alkyl moiety may be liner or branched.

During the preparation of benzophenone glycine imine alkyl ester derivative of general formula (I) according to the present invention, there is no need to use a catalyst to ensure that the yield of product obtained by the process according to the present invention is comparable or even greater than the yield achievable with the processes known from the prior art.

Furthermore, there is no need to use benzophenone imine in that process.

In addition, the organic bases used in the process according to the present invention can be easily recycled in separate work-up methods well known by the man ordinary skilled in the art.

The present invention relates to a process for the preparation of compound of general formula (I) in which Alk represents a C₁-C₆ alkyl moiety. Preferably, Alk represents methyl, ethyl or tertiary butyl.

The present invention relates to a process for the preparation of compound of general formula (I) which is conducted in the presence of a solvent. Preferably, the solvent is an aprotic dipolar solvent. Suitable examples of such solvent may include acetonitrile, propionitrile or butyronitrile.

The present invention relates to a process for the preparation of compound of general formula (I) which is conducted in the presence of a base, in a DCDPM / base molar ratio of from 1 to 5. Preferably, the process may be conducted in the following conditions, chosen alone or in combination :
- the base is a tertiary amine base. Suitable examples of such base may include triethylamine, N-ethyl-diisopropylamine, N,N-dimethylaniline or pyridine; and
- DCDPM / base molar ratio is of from 2 to 3,2.

The present invention relates to a process for the preparation of compound of general formula (I) comprising the reaction of DCDPM with GAE or GAE.HHal in a DCDPM / GAE or GAE.HHal molar ratio of from 0,5 to 2. Preferably, the DCDPM / GAE or GAE.HHal molar ratio is from 1 to 1,2.

Starting from benzophenone glycine imine alkyl ester derivative of formula (I) according to the present invention, 2-aminomethylpyridine derivative might then be prepared in the same way as described in steps (B) to (D) of the process disclosed in International patent application published as WO 2004/065359, herein incorporated by reference.

DCDPM may be easily prepared by reacting benzophenone with PCl₅ or by any other method disclosed in the state of the art and accessible to the man ordinary skilled in the art.

The present invention will now be illustrated with reference to the following examples.

### Example 1: Preparation of benzophenone glycine imine ethyl ester

25,4 g (180 mmol) of glycine ethylester hydrochloride, 99%, 43,1g (180 mmol)of a,a-dichloro-diphenylmethane, 99%, and 300ml of dry acetonitrile were placed in a flask with stirring. 70,2 g (540mmol) of N-ethyl-diisopropylamine were added.

The reactants were stirred at 40°C for 2 hours and then at 70°C for 5 hours (reaction progress was checked by GC-samples). After cooling down to 20°C, 200ml of water were added.

The slurry was extracted with ethyl acetate and the organic extracts were dried with sodium sulphate.

After distilling off the solvent, 47,7g of benzophenone glycine imine ethyl ester remained with purity of 92,9% (GC). Yield was therefore of 92,1% of theory. No further purification was needed.

As a comparison, preparation of benzophenone glycine imine ethyl ester starting from benzophenone in the presence of a catalyst as disclosed in Example 1 of International patent application WO 2004/065359 leads to said compound in a yield of 91%.

### Example 2 : Preparation of benzophenone glycine imine methyl ester

50 g (390mmol) of glycine methylester hydrochloride, 98%, and 122,1g (1,18mol) triethylamine, 98%, were placed in a flask with 200 ml of butyronitrile. The mixture was heated with stirring to 95-100°C and a solution of 93,4g (390mmol) of a,a-dichloro-diphenylmethane and 200 ml of butyronitrile were dropped in within 3 hours.

The reaction was allowed to continue for 1hour at 105°C (GC check). It was cooled to +5°C and stirred for 30 minutes and then the salt was filtered and washed with a small amount of solvent. The filtrates were concentrated in a rotatory evaporator.

105 g (0,352mol) of benzophenone glycine imine methyl ester remained with a purity of 85% (GC) representing a yield of 90,3% of theory. No further purification was needed.

### Example 3 : Preparation of benzophenone glycine imine tertiary butyl ester

Similarly to Example 2, 28,74g (0,12mol) of a,a-dichlorodiphenylmethane,99%(GC) was reacting with 20,74 g (0,12mol) tert.butyl glycine hydrochloride, 97%, and 37,2g (0,36mol) tri ethyl amine, 98%, in 200 ml of butyronitrile.

After work-up, 35,3 g (0,106mol) of benzophenone glycine imine tert. butyl ester remained with a purity of 89% (GC) representing a yield of 88,6% of theory. No further purification was needed

### Example 4 : Preparation of benzophenone glycine imine tertiary butyl ester

Similarly to Example 3, 35,8 g (0,15mol) of a,a-dichlorodiphenylmethane,99%(GC) was reacting, in presence of 2 mol equivalents (24,2g; 0,3mol) of pyridine, 98%, with 20,3g (0,15mol) tert.butyl glycinate, 97%, in 200ml of propionitrile.

After work-up, 44,8 g ( 0,136mol) of benzophenone glycine imine tert. butyl ester remained with a purity of 89,5% (GC) representing a yield of 90,5% of theory. No further purification was needed.

## Claims

1. Process for the preparation of a benzophenone glycine imine alkyl ester derivative of general formula (I) : 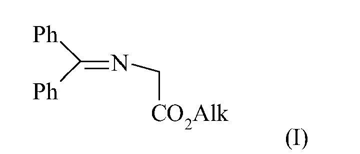 in which :
- Ph represents phenyl; and
- Alk represents a C₁-C₆ alkyl moiety;
according to the following reaction scheme : 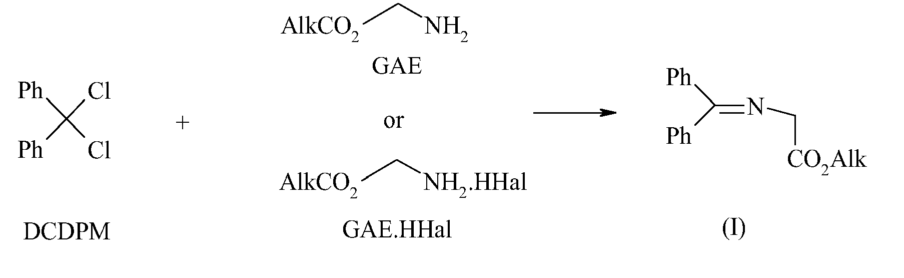 in which a,a-dichloro-diphenylmethane (DCDPM) is reacted with glycine alkyl ester (GAE) or its hydrohalide (GAE.HHal) in a solvent, in the presence of a base, in a DCDPM / GAE or GAE.HHal molar ratio of from 0,5 to 2 and in a DCDPM / base molar ratio of from 1 to 5 to provide a benzophenone glycine imine derivative of general formula (I).

2. A process according to claim 1, **characterised in that** Alk represents methyl, ethyl or tertiary butyl.

3. A process according to claim 1 or 2, **characterised in that** the solvent is an aprotic dipolar solvent.

4. A process according to any of the claims 1 to 3, **characterised in that** the base is a tertiary amine base.

5. A process according to any of the claims 1 to 4, **characterised in that** the DCDPM / base molar ratio is of from 2 to 3,2.

6. A process according to any of the claims 1 to 5, **characterised in that** the DCDPM / GAE or GAE.HHal molar ratio is from 1 to 1,2.
